# EUROPÄISCHE PATENTSCHRIFT

(11) **EP 2 717 885 B1**
(45) Veröffentlichungstag und Bekanntmachung des Hinweises auf die Patenterteilung: **11.08.2021**
(21) Anmeldenummer: 12726137.8
(22) Anmeldetag: 08.06.2012
(51) Int. Cl.: A61K 31/726, A61K 31/727, A61P 35/00, A61P 31/12, A61P 31/22

(54) **ANTIINFEKTIVES MITTEL**
ANTIINFECTIVE COMPOSITION
AGENT ANTI-INFECTIEUX

(30) Priorität: 10.06.2011 DE 102011077393
(43) Veröffentlichungstag der Anmeldung: 16.04.2014
(73) Patentinhaber: Reinmüller, Johannes, 65193 Wiesbaden (DE); Dirting, Kay, 65191 Wiesbaden (DE)
(72) Erfinder: Reinmüller, Johannes, 65193 Wiesbaden (DE); Dirting, Kay, 65191 Wiesbaden (DE)
(74) Vertreter: Weickmann & Weickmann PartmbB
(86) Internationale Anmeldenummer: PCT/EP2012/060942
(87) Internationale Veröffentlichungsnummer: WO 2012/168462

(56) Entgegenhaltungen:
- WO-A1-03/041723
- WO-A1-2005/067944
- WO-A1-2009/102736
- AU-B2- 664 096
- DE-A1- 10 209 966
- US-A- 2 637 724
- JEANNIN JEAN FRANCOIS ET AL: "Antitumor effect of synthetic derivatives of lipid A in an experimental model of colon cancer in the rat", GASTROENTEROLOGY, ELSEVIER, PHILADELPHIA, PA, Bd. 101, Nr. 3, 1. September 1991 (1991-09-01), Seiten 726-733, XP009161740, ISSN: 0016-5085
- PIRNAZAR PAYMAN ET AL: "Bacteriostatic effects of hyaluronic acid", JOURNAL OF PERIODONTOLOGY, AMERICAN ACADEMY OF PERIODONTOLOGY, CHICAGO, IL, US, vol. 70, no. 4, 1 April 1999 (1999-04-01), pages 370-374, XP008162701, ISSN: 0022-3492, DOI: 10.1902/JOP.1999.70.4.370

## Beschreibung

Die vorliegende Erfindung betrifft die Verwendung von Hydroxymethylgruppen-enthaltenden Glycosaminoglycanen, wie insbesondere Hydroxymethyl-Hyaluronsäure, zur Behandlung und Vorbeugung infektiöser bzw. maligner Erkrankungen, insbesondere der Haut oder der Schleimhäute. Gegenstand der Erfindung ist außerdem ein Herstellungsverfahren für mit Hydroxymethylgruppen modifizierte Glycosaminoglycane.

Infektiöse Erkrankungen sind durch Erreger wie Viren, Bakterien oder Pilze hervorgerufene Erkrankungen, die ein breites Spektrum von zeitlichen Verläufen und Symptomen zeigen, die für den Erreger oftmals spezifisch sind. Die Infektion erfolgt durch Kontakt eines Organismus mit einem Krankheitserreger, wobei die Infektionswege charakteristisch für die Erkrankung und deren Erreger sind. Für viele erregerbedingte Krankheiten hält die Medizin spezifische Gegenmittel bereit, wie Antibiotika gegen Bakterien, Antimykotika gegen Pilze und Virostatika gegen Viren. Gegen einige Erreger besteht zudem die Möglichkeit einer vorbeugenden Impfung.

Noch heute sind jedoch manche infektiöse Erkrankungen nicht heilbar. Zudem besteht bei verschiedenen zur Bekämpfung der Erreger eingesetzten Wirkstoffen das Problem einer Resistenzentwicklung. Unter einer Resistenz versteht man die erworbene Widerstandsfähigkeit eines Erregers gegen einen Wirkstoff. Beispielsweise führt bei resistenten Bakterienstämmen die Behandlung mit einem bestimmten oder sogar mehreren Antibiotika nicht mehr zu ihrem gewünschten Absterben oder ihrer Wachstumshemmung. Die Forschung konzentriert sich daher intensiv auch auf die Entwicklung von Therapeutika gegen resistente Erreger.

Eine weitere Problematik stellen Sekundärinfektionen dar, bei denen zusätzlich zu einer zunächst vorhandenen Infektion (Primärinfektion) eine weitere Infektion mit einem anderen Erreger erfolgt. Eine solche nach einer Primärinfektion erfolgte weitere Infektion kann das Immunsystem vor große Probleme stellen und auch die Therapie und Medikation erschweren. Von besonderer Bedeutung sind Sekundärinfektionen mit Viren-, Bakterien- oder Pilzerkrankungen, wenn schon die Primärinfektion mit einer Schwächung des Immunsystems einhergeht. Es wäre daher wünschenswert, Wirkstoffe bereitzustellen, die eine gleichzeitige Bekämpfung unterschiedlicher Erreger ermöglichen.

Jeannin Jean Francois et al. beschreiben in "Antitumor effect of synthetic derivatives of lipid A in an experimental model of colon cancer in the rat" (Gastroenterology, Elsevier, Philadelphia, PA, Bd.101, Nr. 3, September 1991, Seiten 726 - 733) einen Anti-Tumor-Effekt von synthetischen Lipid-A-Derivaten gegenüber Darmkrebs im Rattenmodell.

DE 102 09 966 und WO 2003/041723 offenbaren die Verwendung von pharmazeutischen Zusammensetzungen, die vernetzte oder unvernetzte und vorzugsweise langkettige Hyaluronsäure sowie übliche pharmazeutische Hilfs- oder/und Trägerstoffe enthalten, zur Behandlung viraler Infektionen, insbesondere zur Behandlung von Infektionen mit Herpesviren.

WO 2005/067944 beschreibt die Verwendung von Hyaluronsäure zur Behandlung entzündlicher Erkrankungen, insbesondere Haut- oder Schleimhauterkrankungen.

Vor diesem Hintergrund war es die Aufgabe der vorliegenden Erfindung, eine Möglichkeit zur Behandlung oder Vorbeugung infektiöser Erkrankungen bereitzustellen. Von besonderem Interesse ist in diesem Zusammenhang die Bereitstellung eines Mittels, das für die Behandlung unterschiedlicher infektiöser Erkrankungen eingesetzt werden kann, die durch verschiedene Erreger, wie Viren, Bakterien oder Pilze, verursacht werden.

Überraschenderweise wurde gefunden, dass Glycosaminoglycanderivate, welche mit Hydroxymethylgruppen modifiziert wurden, eine hervorragende antiinfektive Wirkung gegenüber unterschiedlichen Arten von Erregern besitzen. Es stellte sich heraus, dass diese antiinfektive Wirkung auf die Anwesenheit von Hydroxymethylgruppen an dem Glycosaminoglycan zurückzuführen ist.

Ein Gegenstand der Erfindung ist somit ein Hydroxymethylgruppen-enthaltendes Glycosaminoglycan zur Verwendung bei der Behandlung oder Vorbeugung infektiöser bzw. maligner Erkrankungen, wie in den angehängten Ansprüchen definiert.

Glycosaminoglycane sind linear aus sich wiederholenden modifizierten Disacchariden aufgebaute Polysaccharide. Die einzelnen Disaccharideinheiten bestehen dabei aus einer Uronsäure, die 1→3 glykosidisch mit einem Aminozucker, wie N-Acetyl-Glucosamin verbunden ist. Die Disaccharideinheiten der Ketten selbst sind 1→4 glykosidisch verknüpft. Glycosaminoglycane sind Bestandteile vieler biologischer Makromoleküle und bilden das Gerüst vieler faserbildender Stoffe. Beispiele für Glycosaminoglycane sind Hyaluronsäure, Heparin, Chondroitinsulfat, Dermatansulfat und Keratansulfat. Erfindungsgemäß werden außerdem Chitosamin und Poly-N-Acetyl-Glucosamin als Glycosaminoglycane verstanden. Vorzugsweise ist das Glycosaminoglycan eine Hyaluronsäure.

Glycosaminoglycane werden üblicherweise aus proteinhaltigen biologischen Geweben gewonnen. Bekannt ist beispielsweise die Isolierung von Hyaluronsäure aus Hahnenkamm oder Streptokokken. Natürliche Heparine werden unter anderem aus Dünndarmmukosa vom Schwein extrahiert. Chondroitinsulfat wird größtenteils aus Knorpelgewebe von Rindern, Schweinen und Haifischen gewonnen. Außerdem können Glycosaminoglycane aus genetisch veränderten Wirtsorganismen, z.B. Bakterienzellen, erzeugt werden.

In der Medizin werden Hyaluronsäure und Derivate davon zur viskoelasitschen Supplementierung von Gelenken bei Arthrose, zur Auffüllung von Geweben, insbesondere der Dermis, als sogenannte "dermal filler" und zur Behandlung von entzündlichen Erkrankungen der Haut und der Schleimhäute eingesetzt. Beispielsweise beschreibt WO 2005/067944 die Behandlung und Prophylaxe von durch Herpes- und Papillomviren bedingten Erkrankungen der Haut und der Schleimhäute.

Überraschenderweise wurde nun gefunden, dass Glycosaminoglycanderivate, in denen ein oder mehrere Aminogruppen mit Hydroxymethylgruppen substituiert sind, eine antiinfektive Wirkung besitzen und außerdem zur Behandlung maligner Erkrankungen von Körperoberflächen geeignet sind.

Die Disaccharideinheiten, aus welchen Glycosaminoglycane aufgebaut sind, bestehen wie oben erwähnt aus einer Uronsäure und einem Aminozucker. In den erfindungsgemäßen, mit Hydroxymethylgruppen substituierten Glycosaminoglycanderivaten ist an ein oder mehrere Strickstoffatome von Aminogruppen eine Hydroxymethylgruppe angebunden. Die erfindungsgemäßen Hydroxymethylgruppen-enthaltenden Glycosaminoglycane weisen somit charakteristische Substituenten -N(R)-CH₂OH auf, wobei R ein beliebiger Rest, insbesondere H oder Acetyl, sein kann. Diese sind vorzugsweise an Aminozucker der Disaccharideinheiten des Glycosaminoglycans angebunden. Bei Glycosaminoglycanen, die den Aminozucker N-Acetylglucosamin enthalten, ist vorzugsweise die N-Acetylgruppe mit einer Hydroxymethylgruppe substituiert. Charaktertisch für diese Hydroxymethylgruppen-enthaltenden Glycosaminoglycane im Sinne der Erfindung sind -N(Acetyl)-CH₂OH-Gruppen. Diese Verbindungen zeigen überraschenderweise eine besonders hohe antiinfektive Wirkung.

Infektiöse Erkrankungen im Sinne der vorliegenden Erfindung werden durch Herpes- oder Papillomavirus verursacht. Infektiöse Erkrankungen, die nicht durch Papillom- oder Herpesviren verursacht werden, sind auch beschrieben. Infektiöse Erkrankungen, die erfindungsgemäß mittels Hydroxymethylgruppen-enthaltenden Glycosaminoglycanen behandelt oder vorgebeugt werden können, sind infektiöse Haut- oder Schleimhauterkrankungen. Zur Schleimhaut zählen dabei insbesondere auch die Oberflächen des Gastrointestinaltraktes, des Urogenitaltraktes, der Lunge und von Hohlorganen.

Weiterhin wird beschrieben, dass durch die Hydroxymethylgruppen-enthaltenden Gylcosaminoglycane infektiöse Erkrankungen behandelt werden können, bei denen es sich um primär virale Infektionskrankheiten mit nachfolgender Superinfektion durch Bakterien und/oder Pilze handelt, bevorzugt solcher Spezies, die gegen übliche Antibiotika resistent sind. Bei den Viren kommen DNA- und RNA-Viren infrage, bei den Bakterien sind es pathogene gram-negative und gram-positive, anaerob oder aerob wachsende Bakterien, bei den Pilzen sind es bevorzugt Sprosspilze (Hefen) und Strahlenpilze.

Das erfindungsgemäße Mittel eignet sich auch zur Behandlung von malignen Erkrankungen der Körperoberflächen, insbesondere des Basalzellkarzinoms der Epidermis und dessen Vorstufen wie solare Keratose bzw. Morbus Bowen. Dieses wird z.B. mit einer gelförmigen Zubereitung des erfindungsgemäßen Mittels unterspritzt und wächst dann zur Oberfläche, wo es abgestoßen wird. Es wird auch beschrieben, dass andere maligne Erkrankungen adjuvant mit dem erfindungsgemäßen Mittel behandelt werden können, z. B. die Peritonealkarzinose mittels einer in die Bauchhöhle instillierten Lösung.

Bevorzugte Beispiele für erfindungsgemäß verwendete Glycosaminoglycane sind an Aminogruppen mit Hydroxmethyl substituierte Hyaluronsäure, Heparin, Chondroitinsulfat, Chitosamin und Poly-N-Acetyl-Glucosamin, wobei Hyaluronsäure besonders bevorzugt ist.

In den erfindungsgemäßen Glycosaminoglycanderivaten sind ein oder mehrere Aminogruppen mit Hydroxymethylgruppen substituiert. Vorzugsweise liegt der Grad der Hydroxymethylierung im Bereich von 200:1 (0,5 %) bis 1:1 (100%), vorzugsweise 100:1 (1%) bis 10:1 (10 %). Mit zunehmender Hydroxymethylierung der Glycosaminoglycane nimmt überraschenderweise die antiinfektive Wirkung von Glycosaminoglycanen zu. Für Hydroxymethyl-Hyaluronsäure konnte in vitro gezeigt werden, dass insbesondere die virozide Wirkung gesteigert ist. Eine besonders bevorzugte Ausführungsform der Erfindung ist daher Hydroxymethyl-Hyaluronsäure zur Verwendung bei der Behandlung oder Vorbeugung infektiöser Erkrankungen, insbesondere viraler Erkrankungen, die durch Herpes- oder Papillomavirus verursacht sind. Beschrieben sind auch bakteriellef Erkrankungen oder Pilzerkrankungen oder Erkrankungen, die nicht durch Herpes- oder Papillomviren hervorgerufen sind.

Im Vergleich zu bekannten antiinfektiven Mitteln, wie beispielsweise Taurolidin, besitzen die erfindungsgemäßen Hydroxymethylgruppen-enthaltenden Glycosaminoglycane eine verbesserte Gewebeverträglichkeit. Sie verbleiben länger an ihrem Wirkort, wobei die Verweildauer durch die Wahl des Molekulargewichts des verwendeten Glycosaminoglycans sowie dessen Vernetzungsgrad gesteuert werden kann. Beispielsweise bleibt Hydroxymethyl-Hyaluronsäure, abhängig von ihrem Molekulargewicht und Vernetzungsgrad, von etwa 24 Stunden bis zu sechs Monaten am Wirkort.

Ein weiterer Vorteil ist die wesentlich erhöhte Stabilität der Verbindungen, d.h. praktisch keine Dekomposition der Verbindungen unter Abgabe von flüchtigem Formaldehyd. Gegenüber den weit gebräuchlichen antiinfektiven Antibiotika besitzen die erfindungsgemäßen modifizierten Glycosaminoglycane den Vorteil der fehlenden Resistenzentwicklung von Seiten der Erreger und eines erweiterten Erregerspektrums. Abbau und Ausscheidung erfolgen auf natürlichem Wege über spezifische Enzyme.

Zur Behandlung infektiöser Erkrankungen sind Hydroxymethylgruppen-enthaltende Glycosaminoglycane im Sinne der Erfindung sowohl in unvernetzter als auch in vernetzter Form oder als Mischungen geeignet. Besonders bevorzugt werden unvernetzte oder vernetzte Hyaluronsäure oder Mischungen davon verwendet.

Unvernetzte Glycosaminoglycane werden vorzugsweise ausgewählt aus (i) langkettigen Glycosaminoglycanen mit einem durchschnittlichen Molekulargewicht (Gewichtsmittel) von mindestens 200 kD und (ii) kurzkettigen Glycosaminoglycanen mit einem durchschnittlichen Molekulargewicht (Gewichtsmittel) von bis zu 50 kD oder Mischungen davon.

Vernetzte Glycosaminoglycane können z.B. kovalent oder nicht kovalent vernetzt sein. Die Herstellung der vernetzten Glycosaminoglycane kann an sich auf bekannte Weise erfolgen. Die kovalente Vernetzung erfolgt dabei im Allgemeinen durch Vernetzung mit bifunktionellen reaktiven Agentien, wie zum Beispiel Diepoxyoktan, BDDE, Divinylsulfon, Glutaraldehyd oder Carbodiimid, über bifunktionelle Aminosäuren, z.B. Lysin, Protamin oder Albumin. Es können z.B. aber auch Vernetzungen über eine Amid-, Ester- oder Etherbindung hergestellt werden. Weitere geeignete Reagenzien zur kovalenten Vernetzung von Glycosaminoglycanen sind Ethylenglycol- oder 1-4-Butandiol-diglycidether, Divinylsulfon, Photoquervernetzungsreagentien wie Ethyleosin, Hydrazide wie Bishydrazid, Trishydrazid und polyvalente Hydrazidverbindungen. Weiterhin können auch intra- und/oder intermolekular veresterte oder mit Hexamethylendiamin vernetzte Glycosaminoglycane eingesetzt werden. Besonders bevorzugt ist eine nicht kovalente Quervernetzung unter Verwendung mehrwertiger Metallionen, wie etwa Eisen, Kupfer, Zink, Calcium, Magnesium, Mangan, Barium und anderen chelatierenden Metallionen.

Bei der Anwendung ist von Bedeutung das Molekulargewicht, sowie bei vernetzten Glycosaminoglycanen der Vernetzungsgrad, der beispielsweise im Bereich von 0,1 bis 10 % liegt, ohne darauf begrenzt zu sein. Generell ist festzustellen, dass bei langkettigen Glycosaminoglycanen ein geringerer Vernetzungsgrad ausreicht, um eine gelartige Matrix zu erhalten, während bei kurzkettigen Glycosaminoglycanen ein höherer Vernetzungsgrad erforderlich ist, um vergleichbare Eigenschaften zu erhalten.

Das erfindungsgemäße Glycosaminoglycan kann sowohl in der Humanmedizin als auch in der Veterinärmedizin, beispielsweise zur Behandlung von Haustieren oder Nutztieren, eingesetzt werden.

Die Verabreichung des Hydroxymethylgruppen-enthaltenden Glycosaminoglycans kann grundsätzlich auf beliebige Art und Weise erfolgen, sofern diese zur Behandlung der jeweiligen Erkrankung geeignet ist. Es ist eine systemische oder lokale Verabreichung denkbar. In vielen Fällen erfolgt eine lokale Verabreichung im Bereich der erkrankten Körperstelle.

Ein erfindungsgemäßes Hydroxymethylgruppen-enthaltendes Glycosaminoglycan kann zur Verwendung bei der Behandlung infektiöser Erkrankungen in Form einer pharmazeutischen Zusammensetzung vorliegen, in der ein oder mehrere Hydroxymethylgruppen-enthaltende Glycosaminoglycane in einer Menge von vorzugsweise 0,01 bis 20 Gewichtsprozent, bezogen auf die gesamte pharmazeutische Zusammensetzung, insbesondere in einer Menge von 0,01 bis 5 Gewichtsprozent und besonders bevorzugt in einer Menge von 0,01 bis 1 Gewichtsprozent enthalten sind.

Als pharmazeutische Hilfsstoffe können die erfindungsgemäßen pharmazeutischen Zusammensetzungen, z.B. Mittel zur pH-Wert-Einstellung, Stabilisierungsmittel, Antioxidantien, Lösungsvermittler, penetrationsfördernde Mittel, Konservierungsmittel oder/und Gelbildner enthalten, wie sie in derartigen Zusammensetzungen üblicherweise verwendet werden. Sie werden in den in derartigen Zubereitungen üblichen Mengen verwendet.

Die erfindungsgemäßen pharmazeutischen Zusammensetzungen können gegebenenfalls neben dem Hydroxymethylgruppen-enthaltenden Glycosaminoglycan auch noch ein oder mehrere weitere pharmazeutische Wirkstoffe enthalten, die mit dem Glycosaminoglycanderivat verträglich sind. Beispiele für weitere Wirkstoffe sind Wirkstoffe zur Therapie von Hauterkrankungen (Dermatosen), Antimykotika, Antibiotika (z.B. Gentamycin, Vancomyzin, Penicillin oder Cephalosporin), Sulfonamide, Desinfektionsmittel, Hormone (z.B. Corticoide) und Hormonabkömmlinge (z.B. Cortisol), lokale Anästhetika (z.B. vom Typ des Lidocains oder Novocains), vasoaktive Substanzen zur Gefäßkonstriktion (Vermeidung von Blutungen), Adrenalin, Enzyme (wie z.B. Hyaluronidase), Interleukine, Wachstumsfaktoren (z.B. EGF, PDGF oder/und IGF), Vitamine (z.B. Vitamin D), Hautpflegemittel und/oder durchblutungsfördernde (hyperämisierende) Mittel. Die weiteren Wirkstoffe können gegebenenfalls mit dem Glycosaminoglycan assoziiert sein, z.B. durch kovalente oder nicht kovalente Wechselwirkungen.

Von Bedeutung sind auch Zusatzstoffe, wie z.B. 2- oder 3-wertige Metallionen, die durch Gelatbildung quervernetzend und stabilisierend wirken können, die andererseits auch den Abbau der wirksamen Glycosaminoglycane beschleunigen können.

Im Gewebe erfolgt der Abbau von Glycosaminoglycanen natürlicherweise durch eine Vielzahl unterschiedlicher Enzyme oder durch Sauerstoffradikale. Hyaluronsäure wird durch Hyaluronidasen oder Sauerstoffradikale abgebaut. Daher sind weiterhin von Bedeutung Zusatzstoffe, die hemmend auf Enzyme wie Hyaluronidase einwirken (Heparin, Indometazin oder/und Salicylate) und solche, die den oxidativen Abbau im Gewebe als sogenannte Radikalfänger verhindern (Vitamine A, E oder/und C).

In einer besonders bevorzugten Ausführungsform der Erfindung wird zur Behandlung oder Vorbeugung infektiöser Erkrankungen eine Mischung aus langkettigen Glycosaminoglycanen (≥ 200 kD) mit kurzkettigen Glycosaminoglycanen (z.B. Hexamere der repetitiven Disaccharideinheiten oder größere Einheiten bis zu 50 kD) oder auch Mischungen der vorgenannten mit quervernetzten Glycosaminoglycanen verwendet. Durch die Mischung entstehen viskose injizierbare Zubereitungen, die bevorzugt mit Injektionskanülen intradermal oder an die Grenze des dermoepidermalen Übergangs durch Injektion flächenhaft eingebracht werden. Auch das Setzen einzelner Quaddeln ist erfindungsgemäß geeignet. Den injizierbaren Glycosaminoglycan-Zubereitungen können zur Minimierung der Schmerzhaftigkeit des Einstichs die bekannten Lokalanästhetika zugesetzt werden.

Eine weitere besonders bevorzugte Ausführungsform sind Mischungen aus quervernetzten und nicht quervernetzten Glycosaminoglycanen.

Anstelle der Injektionstechnik mit Kanülen lassen sich die erfindungsgemäßen Zubereitungen äußerst wirksam auch durch Druckinjektion anwenden. Dieses Verfahren zeichnet sich durch weitgehende Schmerzfreiheit aus.

Weitere erfindungsgemäße Zubereitungen des Wirkstoffs sind wässrige Lösungen oder Emulsionen zur intravenösen Applikation oder zur Instillation in Körperhöhlen oder Hohlorgane.

Weiterhin können die erfindungsgemäßen Zubereitungen auch topisch, d.h. oberflächlich auf die Haut in Form von Salben, Cremes, Lotionen, Gelen, Sprays, Tinkturen, Shampoos, Oklusivfolien aufgebracht werden. Eine besondere Zubereitungsform im Sinne der Erfindung ist eine trockene Glycosaminoglycan-Zubereitung in Form eines Puders, welches insbesondere zur Behandlung nässender Ekzeme Verwendung findet. Auch das Einbringen der Wirkstoffe in mikroverkapselter Form bzw. in Form von Liposomen ist Gegenstand der Erfindung. Soweit Schleimhäute der Atemwege von einer infektiösen Erkrankung betroffen sind, kann eine Behandlung mit den erfindungsgemäßen Hydroxymethylgruppen-enthaltenden Glycosaminoglycanen auch unter Anwendung eines Aerosols als Inhalationslösung erfolgen.

Die Herstellung einer erfindungsgemäßen Zusammensetzung kann auf eine, für die Herstellung derartiger Zusammensetzungen an sich übliche, allgemein bekannte Weise, erfolgen. Dabei ist die Reihenfolge der Vermischung der einzelnen Bestandteile in der Regel nicht kritisch.

Die Art, Dosis und Häufigkeit der Verabreichung der erfindungsgemäßen Zusammensetzung sowie die Beschaffenheit (z.B. Viskosität, Vernetzungsgrad, Wirkstoffgehalt etc.) richten sich insbesondere nach der Art und Schwere der Erkrankung sowie nach dem Alter des Patienten und dem Ort und der Art der Applikation, z.B. dem Zustand und der Empfindlichkeit der betroffenen Körperstellen. Werden die erfindungsgemäßen Zusammensetzungen in Form topisch applizierbarer Zubereitungen verabreicht, so entspricht die Verabreichung in der Regel den für derartige Zusammensetzungen üblichen Bedingungen.

Die Art der Behandlung und die Häufigkeit der Applikation richtet sich insbesondere auch nach dem individuellen Ansprechen der zu behandelnden Personen. Vorzugsweise erfolgt eine Applikation von Gelen oder Lösungen in Abständen von mehreren Tagen bis zu 1 oder 2 Monaten, insbesondere ca. 1-2 Wochen.

Die Erfindung beinhaltet auch Mischungen eines Hydroxymethylgruppen-enthaltenden Glycosaminoglycans mit anderen Glycosaminoglycanen in vernetzter oder/und nicht vernetzter Form. Zu möglichen Kombinationen und Quervernetzungsmöglichkeiten wird Bezug genommen auf EP-B-0 619 737, DE-A-102 99 66 und WO 03/041723. Mischungen aus Hydroxymethylgruppen-enthaltender Hyaluronsäure und Heparin sind bevorzugt. Weiterhin bevorzugt sind Mischungen aus Hydroxymethylgruppen-enthaltender Hyaluronsäure und positiv geladenen Glycosaminoglycanen wie Chitosamin.

Die Herstellung eines Hydroxymethylgruppen-enthaltenden Glycosaminoglycans umfasst vorzugsweise die Schritte:
(i) Bereitstellen von Glycosaminoglycan und
(ii) Substituieren ein oder mehrerer Aminogruppen des Glycosaminoglycans mit Hydroxymethylgruppen.

Als Glycosaminoglycan Ausgangsprodukt in Schritt (i) wird vorzugsweise Hyaluronsäure, Heparin, Chondroitinsulfat, Chitosamin oder Poly-N-Acetylglucosamin verwendet.

In einer besonders bevorzugten Ausführungsform ist das Glycosaminoglycan in Schritt (i) aus einer biologischen Quelle isoliert oder biotechnologisch gewonnen, insbesondere aus Hahnenkamm oder Bakterien, wie B. *subtilis* oder Streptokokken-Kulturen.

Die Gewinnung des Glycosaminoglycans aus einer biologischen Quelle oder aus Bakterien kann in Anwesenheit von Formaldehyd oder einem anderen Hydroxymethylgruppendonor erfolgen. Das gereinigte Glycosaminoglycan aus Schritt (i) liegt dann beispielsweise als wässrige Lösung, als Präzipitat oder als Hydrogel vor.

In Schritt (ii) wird das Glycosaminoglycan dann durch chemische Behandlung mit Hydroxymethylgruppen substituiert. Schritt (ii) kann zusammen mit Schritt (i) oder danach durchgeführt werden. Schritt (ii) kann beispielsweise die Umsetzung des Glycosaminoglycans mit Formaldehyd oder einem unter den Reaktionsbedingungen Formaldehyd freisetzenden Mittel, wie etwa Taurolidin, umfassen. Ein bevorzugtes Verfahren zur Einführung von Hydroxymethylgruppen in das Glycosaminoglycan (z.B. Hyaluronsäure) verwendet Taurolidin in 1-2 %iger Lösung. Glycosaminoglycan wird dabei mit der Taurolidinlösung im Verhältnis 1 mg auf 1 ml versetzt und für 24 h unter Verschluss oder unter einer Gasatmosphäre von 1-100 bar inkubiert. Vorteil dieses Vorgehens ist, dass Taurolidin selbst sehr viel weniger toxisch ist als Formaldehyd.

Anschließend kann das mit Hydroxymethylgruppen modifizierte Glycosaminoglycan in einem weiteren Schritt (iii) aufgereinigt werden. Überschüssiges Formaldehyd bzw. Reste von Formaldehyd-freisetzenden Reagenzien aus Schritt (ii) werden dabei entfernt. Die Aufreinigung kann beispielsweise durch Fällen mit z.B. Alkoholen oder Salzen, durch Chromatographieverfahren, Dialyseverfahren, Vakuumextraktion und/oder Gefriertrocknen erfolgen.

Optional erfolgt anschließend ein weiterer Schritt (iv), in dem das mit Hydroxymethylgruppen substituierte Glycosaminoglycan quervernetzt wird. Grundsätzlich kann auch zuerst die Quervernetzung vorgenommen werden und dann die Einführung von Hydroxymethylgruppen. Die Quervernetzung kann, wie vorstehend beschrieben, nach im Stand der Technik bekannten Verfahren erfolgen. In einer besonders bevorzugten Ausführungsform erfolgt in Schritt (iv) eine Quervernetzung mit ein oder mehreren weiteren Glycosaminoglycanen, die optional selbst an Aminogruppen mit Hydroxymethylgruppen substituiert sein können. Beispiele für solche weiteren optional mit Hydroxymethylgruppen modifizierten Glycosaminoglycane sind Heparin, Chondroitinsulfat, Chitosamin und Poly-N-Acetylglucosamin.

In einer weiteren bevorzugten Ausführungsform der Erfindung wird ein Hydroxymethylgruppen-enthaltendes Glycosaminoglycan anschließend mit ein oder mehreren weiteren Wirkstoffen und/oder Zusatzstoffen kombiniert. Beispiele für solche weiteren Wirkstoffe und Zusatzstoffe sind vorstehend erläutert.

Ein weiterer Aspekt der Erfindung betrifft eine Kombination von Hydroxymethylgruppen-enthaltendem Glycosaminoglycan mit Taurolidin, z.B. mit einer Taurolidin-Lösung, z.B. einer 1-2 % (w/v) Taurolidinlösung. Als Glycosaminoglycan wird dabei vorzugsweise Hyaluronsäure verwendet, wobei das Molekulargewicht der Hyaluronsäure beispielsweise zwischen 100.000 und 10.000.000 Dalton liegen kann.

Eine erfindungsgemäße Zusammensetzung kann vorzugsweise in gasdichter Verpackung, wie z.B. einer Glasspritze, gelagert werden und zeichnet sich durch eine erhöhte antiinfektive und prolongierte Wirkung sowie durch eine verbesserte Gewebeverträglichkeit aus.

Weiterhin wird ein Verfahren zur Behandlung einer bakteriellen Entzündung des Kniegelenks beschrieben, wobei das Hydroxmethylgruppen-enthaltende Glycosaminoglycan nach einem der Ansprüche 1-8, bevorzugt als Gel instilliert oder als Spüllösung eingesetzt wird.

### Anwendungsbeispiele

### Beispiel 1

Fermentativ gewonnene HA aus *Streptococcus equi* wird in Taurolidin-Lösung 2% gelöst und 12 Std. bei 40 °C gehalten. Danach wird überschüssige Flüssigkeit durch Filtration abgeschieden und verbliebene Flüssigkeit durch Vakuumtrocknung entfernt. Das erhaltene Produkt wird dann in physiologischer NaCI-Lösung gelöst und der pH-Wert der Lösung wird mit 1 % NaOH auf 10 eingestellt. Sodann wird BDDE zugesetzt bis zu einer Konzentration von 0,2 %. Der Ansatz wird bei 40 °C für 4 Std. inkubiert. Danach wird mit Na-Acetat der pH-Wert des Ansatzes auf 7,4 eingestellt und dieser für weitere 24 Std. bei 70 Grad Celsius gehalten. Nach Abkühlung auf Raumtemperatur kann die Weiterverarbeitung des Ansatzes zum antiinfektiven Endprodukt, z.B. Augengel, erfolgen.

### Beispiel 2

*Streptococcus equi* Spezies werden in Fermentatoren in einer CO₂ angereicherten anaeroben Atmosphäre gezüchtet. Nach Beendigung des Wachstums bzw. der Vermehrung wird der Nährlösung Formaldehyd in konzentrierter Form zugegeben, sodass die Bakterien absterben.

Die Lösung wird sodann zentrifugiert und der Überstand abgezogen. Das erhaltene Material wird mit verdünnter NaOH versetzt und für 4 Std. geschüttelt. Die Lösung wird danach neutralisiert und ultrafiltiert. Die erhaltene Lösung kann mit Alkohol behandelt und die gelöste HA ausgefällt und getrocknet werden. Es liegt damit ein erfindungsgemäßes Produkt vor, welches je nach beabsichtigter Anwendung weiter verarbeitet werden kann.

### Beispiel 3

100 ml einer 2 %igen Hyaluronsäurelösung werden mit 100 ml einer 2 %igen Taurolinlösung gemischt und unter Luftabschluss in einer Glasflasche aufbewahrt. Die Lösung kann bei Bedarf über ein Schlauch- bzw. Kathetersystem in die Bauchhöhle oder in die Harnblase instilliert werden.

## Patentansprüche

1. Hydroxymethylgruppen-enthaltendes Glycosaminoglycan, worin eine oder mehrere Aminogruppen mit Hydroxymethyl substituiert sind, zur Verwendung bei der Behandlung oder Vorbeugung viraler infektiöser bzw. maligner Haut- oder Schleimhauterkrankungen, wobei die viralen infektiösen Erkrankungen durch Herpes- oder Papillomviren verursacht sind, und wobei die malignen Erkrankungen Basalzellkarzinom der Epidermis und dessen Vorstufen sind, und wobei Glycosaminoglycane linear aus sich wiederholenden modifizierten Disacchariden aufgebaute Polysaccharide sind, wobei die Disaccharideinheiten der Ketten selbst 1→4 glykosidisch verknüpft sind.

2. Hydroxymethylgruppen-enthaltendes Glycosaminoglycan nach Anspruch 1 zur Verwendung nach Anspruch 1,
**dadurch gekennzeichnet,**
**dass** die Vorstufen des Basalzellkarzinoms der Epidermis solare Keratose und Morbus Bowen sind.

3. Hydroxymethylgruppen-enthaltendes Glycosaminoglycan nach Anspruch 1 oder 2 zur Verwendung nach Anspruch 1 oder 2,
**dadurch gekennzeichnet,**
**dass** das Glycosaminoglycan ausgewählt ist aus Hyaluronsäure, Heparin, Chondroitinsulfat, Chitosamin und Poly-N-Acetyl-Glucosamin, vorzugsweise Hyaluronsäure worin jeweils eine oder mehrere Aminogruppen mit Hydroxmethyl substituiert sind.

4. Hydroxymethylgruppen-enthaltendes Glycosaminoglycan nach einem der vorhergehenden Ansprüche zur Verwendung nach einem der vorhergehenden Ansprüche,
**dadurch gekennzeichnet,**
**dass** der Grad der Hydroxymethylierung im Bereich von 200:1 (0,5%) bis 1:1 (100%), vorzugsweise 100:1 (1%) bis 10:1 (10%) liegt.

5. Hydroxymethylgruppen-enthaltendes Glycosaminoglycan nach einem der vorhergehenden Ansprüche zur Verwendung nach einem der vorhergehenden Ansprüche,
zur lokalen Verabreichung, insbesondere zur intradermalen Verabreichung, zur Verabreichung an der Grenze des dermo-epithelialen Übergangs oder zur topischen Verabreichung.

6. Hydroxymethylgruppen-enthaltendes Glycosaminoglycan nach einem der vorhergehenden Ansprüche zur Verwendung nach einem der vorhergehenden Ansprüche,
**dadurch gekennzeichnet,**
(a) **dass** das Hydroxymethylgruppen-enthaltende Glycosaminoglycan in unvernetzter Form vorliegt, insbesondere ausgewählt aus
(i) langkettigen Glycosaminoglycanen mit einen durchschnittlichen Molekulargewicht (Gewichtsmittel) von mindestens 200 kD und
(ii) kurzkettigen Glycosaminoglycanen mit einen durchschnittlichen Molekulargewicht (Gewichtsmittel) bis zu 50 kD
oder Mischungen davon, oder
(b) **dass** das Hydroxymethylgruppen-enthaltende Glycosaminoglycan in vernetzter Form vorliegt, insbesondere dass der Vernetzungsgrad im Bereich von 0,1% bis 10% liegt, oder
(c) **dass** das Hydroxymethylgruppen-enthaltende Glycosaminoglycan als eine Mischung unvernetzter und vernetzter Glycosaminoglycane vorliegt.

7. Hydroxymethylgruppen-enthaltendes Glycosaminoglycan nach einem der vorhergehenden Ansprüche zur Verwendung nach einem der Ansprüche 1 bis 6,
**dadurch gekennzeichnet,**
**dass** es als injizierbare Zubereitung, Salbe, Creme, Lotion, Gel, Spray, Tinktur, Spül- oder Infusionslösung, Tropfenlösung, Shampoo, Okklusivfolie, Puder oder inhalierbare Zubereitung, insbesondere Aerosol, vorliegt.

8. Hydroxymethylgruppen-enthaltendes Glycosaminoglycan nach einem der vorhergehenden Ansprüche zur Verwendung nach einem der Ansprüche 1 bis 7,
**dadurch gekennzeichnet,**
**dass** die infektiöse Haut- oder Schleimhauterkrankung ausgewählt ist aus Erkrankungen von Körperoberflächen, des Gastrointestinaltrakts, des Knies, des Urogenitaltrakts und der Lunge.

9. Hydroxymethylgruppen-enthaltendes Glycosaminoglycan nach einem der vorhergehenden Ansprüche zur Verwendung nach einem der vorhergehenden Ansprüche,
in der Human- oder Veterinärmedizin.

10. Hydroxymethylgruppen-enthaltendes Glycosaminoglycan nach einem der vorhergehenden Ansprüche zur Verwendung nach einem der vorhergehenden Ansprüche,
**dadurch gekennzeichnet,**
**dass** das Hydroxymethylgruppen-enthaltende Glycosaminoglycan in Kombination mit mindestens einem Hemmstoff des Glycosaminoglycanabbaus vorliegt, wobei der Hemmstoff des Glycosaminoglycanabbaus insbesondere ausgewählt ist aus Heparin, Indomethazin, Salicylaten, Radikalfängern, wie Vitamin A, C oder E, und Mischungen davon.

11. Hydroxymethylgruppen-enthaltendes Glycosaminoglycan nach einem der vorhergehenden Ansprüche zur Verwendung nach einem der vorhergehenden Ansprüche,
**dadurch gekennzeichnet,**
**dass** das Hydroxymethylgruppen-enthaltende Glycosaminoglycan in Kombination mit ein oder mehreren weiteren Glycosaminoglycanen in vernetzter oder unvernetzter Form vorliegt, welche optional Hydroxymethylgruppen enthalten können, wobei die weiteren Glycosaminoglycane insbesondere ausgewählt sind aus Heparin, Chondroitinsulfat, Chitosamin, Poly-N-Acetyl-Glucosamin.

12. Hydroxymethylgruppen-enthaltendes Glycosaminoglycan nach einem der vorhergehenden Ansprüche, zur Verwendung insbesondere nach einem der vorhergehenden Ansprüche,
**dadurch gekennzeichnet,**
**dass** das Hydroxymethylgruppen-enthaltende Glycosaminoglycan in Kombination mit Taurolidin vorliegt.

13. Hydroxymethylgruppen-enthaltendes Glycosaminoglycan nach einem der vorhergehenden Ansprüche zur Verwendung nach einem der vorhergehenden Ansprüche in der Behandlung oder Vorbeugung von infektiösen Erkrankungen des Auges, insbesondere der Bindehaut oder Hornhaut des Auges wie etwa Bindehautentzündung,
wobei das Hydroxymethylgruppen-enthaltende Glycosaminoglycan nach einem der Ansprüche 1-7, bevorzugt als Gel instilliert oder als Spüllösung eingesetzt wird.

14. Verfahren zur Herstellung eines Hydroxymethylgruppen-enthaltenden Glycosaminoglycans, umfassend die Schritte:
(i) Bereitstellen von Glycosaminoglycan und
(ii) Substituieren ein oder mehrerer Aminogruppen des Glycosaminoglycans mit Hydroxymethylgruppen,
wobei das Glycosaminoglycan insbesondere ausgewählt ist aus Hyaluronsäure, Heparin, Chondroitinsulfat, Chitosamin und Poly-N-Acetyl-Glucosamin.

15. Verfahren nach Anspruch 14,
(a) wobei das Glycosaminoglycan in Schritt (i) aus einer biologischen Quelle isoliert oder biotechnologisch gewonnen wird, insbesondere aus Hahnenkamm oder transgenen Bakterien wie *B. subtilis* oder Streptokokken-Kulturen, und/oder
(b) wobei Schritt (ii) die Umsetzung des Glycosaminoglycans mit Formaldehyd oder einem unter den Reaktionsbedingungen Formaldehyd freisetzenden Mittel wie Taurolidin umfasst, und/oder
(c) weiterhin umfassend den Schritt
(iii) Aufreinigen des mit Hydroxymethylgruppen substituierten Glycosaminoglycans, insbesondere durch Fällen mit z.B. Alkoholen oder Salzen, Chromatographieverfahren, Dialyseverfahren, Vakuumextraktion oder Gefriertrocknen, und/oder
(d) weiterhin umfassend den Schritt
(iv) Quervernetzen des mit Hydroxymethylgruppen substituierten Glycosaminoglycans, wobei Schritt (iv) insbesondere das Quervernetzen mit ein oder mehreren anderen Glycosaminoglycanen umfasst, die selbst ebenfalls an Aminogruppen mit Hydroxymethylgruppen modifiziert sein können.

## Claims

1. Hydroxymethyl-group-containing glycosaminoglycan in which one or more amino groups are substituted with hydroxymethyl, for use in the treatment or prevention of viral infectious or malignant skin or mucosal diseases, wherein the viral infectious diseases are caused by herpes viruses or papillomaviruses, and wherein the malignant diseases are basal cell carcinoma of the epidermis and its precursors, and wherein glycosaminoglycans are linear polysaccharides composed of repeating modified disaccharides, wherein the disaccharide units of the chains are themselves linked 1→4 glycosidically.

2. Hydroxymethyl-group-containing glycosaminoglycan according to claim 1 for use according to claim 1,
**characterised in that**
the precursors of basal cell carcinoma of the epidermis are solar keratosis and Bowen's disease.

3. Hydroxymethyl-group-containing glycosaminoglycan according to either claim 1 or claim 2 for use according to either claim 1 or claim 2,
**characterised in that**
the glycosaminoglycan is selected from hyaluronic acid, heparin, chondroitin sulfate, chitosamine and poly-N-acetyl glucosamine, preferably hyaluronic acid in which one or more amino groups are substituted with hydroxymethyl.

4. Hydroxymethyl-group-containing glycosaminoglycan according to any of the preceding claims for use according to any of the preceding claims,
**characterised in that**
the degree of hydroxymethylation is in the range from 200:1 (0.5%) to 1:1 (100%), preferably 100:1 (1%) to 10:1 (10%).

5. Hydroxymethyl-group-containing glycosaminoglycan according to any of the preceding claims for use according to any of the preceding claims,
for local administration, in particular for intradermal administration, for administration at the border of the dermo-epithelial junction or for topical administration.

6. Hydroxymethyl-group-containing glycosaminoglycan according to any of the preceding claims for use according to any of the preceding claims,
**characterised in that**
(a) the hydroxymethyl-group-containing glycosaminoglycan is present in uncrosslinked form, in particular selected from
(i) long-chain glycosaminoglycans having an average molecular weight (weight average) of at least 200 kD and
(ii) short-chain glycosaminoglycans having an average molecular weight (weight average) of up to 50 kD
or mixtures thereof, or
(b) **in that** the hydroxymethyl-group-containing glycosaminoglycan is present in crosslinked form, in particular **in that** the degree of crosslinking is in the range from 0.1% to 10%, or
(c) **in that** the hydroxymethyl-group-containing glycosaminoglycan is present as a mixture of uncrosslinked and crosslinked glycosaminoglycans.

7. Hydroxymethyl-group-containing glycosaminoglycan according to any of the preceding claims for use according to any of claims 1 to 6,
**characterised in that**
it is present as an injectable preparation, ointment, cream, lotion, gel, spray, tincture, rinsing or infusion solution, drop solution, shampoo, occlusive film, powder or inhalable preparation, in particular an aerosol.

8. Hydroxymethyl-group-containing glycosaminoglycan according to any of the preceding claims for use according to any of claims 1 to 7,
**characterised in that**
the infectious skin or mucosal disease is selected from diseases of body surfaces, the gastrointestinal tract, the knee, the urogenital tract and the lungs.

9. Hydroxymethyl-group-containing glycosaminoglycan according to any of the preceding claims for use according to any of the preceding claims,
in human or veterinary medicine.

10. Hydroxymethyl-group-containing glycosaminoglycan according to any of the preceding claims for use according to any of the preceding claims,
**characterised in that**
the hydroxymethyl-group-containing glycosaminoglycan is present in combination with at least one inhibitor of glycosaminoglycan degradation, the inhibitor of glycosaminoglycan degradation being selected in particular from heparin, indometazine, salicylates, radical scavengers, such as vitamin A, C or E, and mixtures thereof.

11. Hydroxymethyl-group-containing glycosaminoglycan according to any of the preceding claims for use according to any of the preceding claims,
**characterised in that**
the hydroxymethyl-group-containing glycosaminoglycan is present in combination with one or more further glycosaminoglycans in crosslinked or uncrosslinked form, which further glycosaminoglycans can optionally contain hydroxymethyl groups, the further glycosaminoglycans being selected in particular from heparin, chondroitin sulfate, chitosamine and poly-N-acetyl glucosamine.

12. Hydroxymethyl-group-containing glycosaminoglycan according to any of the preceding claims, for use in particular according to any of the preceding claims,
**characterised in that**
the hydroxymethyl-group-containing glycosaminoglycan is present in combination with taurolidine.

13. Hydroxymethyl-group-containing glycosaminoglycan according to any of the preceding claims for use according to any of the preceding claims in the treatment or prevention of infectious diseases of the eye, in particular of the conjunctiva or cornea of the eye such as conjunctivitis,
wherein the hydroxymethyl-group-containing glycosaminoglycan according to any of claims 1-7 is preferably instilled as a gel or used as a rinsing solution.

14. Method for producing a hydroxymethyl-group-containing glycosaminoglycan, comprising the steps of:
(i) providing glycosaminoglycan and
(ii) substituting one or more amino groups of the glycosaminoglycan with hydroxymethyl groups,
the glycosaminoglycan being selected in particular from hyaluronic acid, heparin, chondroitin sulfate, chitosamine and poly-N-acetyl glucosamine.

15. Method according to claim 14,
(a) wherein the glycosaminoglycan in step (i) is isolated from a biological source or obtained biotechnologically, in particular from cockscomb or transgenic bacteria such as *B. subtilis* or streptococcal cultures, and/or
(b) wherein step (ii) comprises reacting glycosaminoglycan with formaldehyde or an agent which releases formaldehyde under the reaction conditions, such as taurolidine, and/or
(c) further comprising the step of
(iii) purifying the hydroxymethyl-group-substituted glycosaminoglycan, in particular by precipitation using e.g. alcohols or salts, chromatography processes, dialysis processes, vacuum extraction or freeze-drying, and/or
(d) further comprising the step of
(iv) crosslinking the hydroxymethyl-group-substituted glycosaminoglycan, wherein step (iv) in particular comprises crosslinking with one or more other glycosaminoglycans, which themselves can also be modified with hydroxymethyl groups at amino groups.

## Revendications

1. Glycosaminoglycane contenant des groupes hydroxyméthyle, dans lequel un ou plusieurs groupes amino sont substitués par de l'hydroxyméthyle, destiné à être utilisé lors du traitement ou de la prophylaxie de maladies de la peau ou des muqueuses virales infectieuses ou malignes, dans lequel les maladies virales infectieuses sont provoquées par des virus herpétiques ou des papillomavirus, et dans lequel les maladies malignes sont un carcinome basocellulaire de l'épiderme et ses stades préliminaires, et dans lequel des glycosaminoglycanes sont des polysaccharides produits de manière linéaire à partir de disaccharides récurrents modifiés, dans lequel les motifs disaccharides des chaînes elles-mêmes 1→4 sont combinés de manière glycosidique.

2. Glycosaminoglycane contenant des groupes hydroxyméthyle selon la revendication 1 destiné à être utilisé selon la revendication 1,
**caractérisé en ce**
**que** les stades préliminaires du carcinome basocellulaire de l'épiderme sont des kératoses actiniques et des maladies de Bowen.

3. Glycosaminoglycane contenant des groupes hydroxyméthyle selon la revendication 1 ou 2 destiné à être utilisé selon la revendication 1 ou 2,
**caractérisé en ce**
**que** le glycosaminoglycane est choisi parmi l'acide hyaluronique, l'héparine, le sulfate de chondroïtine, la chitosamine, et la poly-N-acétylglucosamine, de préférence l'acide hyaluronique, dans lequel respectivement un ou plusieurs groupes amino sont substitués par de l'hydroxyméthyle.

4. Glycosaminoglycane contenant des groupes hydroxyméthyle selon l'une quelconque des revendications précédentes destiné à être utilisé selon l'une quelconque des revendications précédentes,
**caractérisé en ce**
**que** le degré de l'hydroxyméthylation se situe dans la plage de 200:1 (0,5 %) à 1:1 (100 %), de préférence de 100:1 (1%) à 10:1 (10%).

5. Glycosaminoglycane contenant des groupes hydroxyméthyle selon l'une quelconque des revendications précédentes destiné à être utilisé selon l'une quelconque des revendications précédentes,
destiné à l'administration locale, en particulier à l'administration intradermique, destiné à l'administration à la limite de la jonction dermo-épidermique ou destiné à l'administration topique.

6. Glycosaminoglycane contenant des groupes hydroxyméthyle selon l'une quelconque des revendications précédentes destiné à être utilisé selon l'une quelconque des revendications précédentes,
**caractérisé en ce**
(a) **que** le glycosaminoglycane contenant des groupes hydroxyméthyle est présent sous une forme non réticulée, en particulier est choisi parmi
(i) des glycosaminoglycanes à chaîne longue avec un poids moléculaire moyen (poids moyen) d'au moins 200 kD, et
(ii) des glycosaminoglycanes à chaîne courte avec un poids moléculaire moyen (poids moyen) jusqu'à 50 kD
ou des mélanges de ceux-ci, ou
(b) **que** le glycosaminoglycane contenant des groupes hydroxyméthyle est présent sous une forme réticulée, en particulier que le degré de réticulation se situe dans la plage de 0,1 % à 10%, ou
(c) **que** le glycosaminoglycane contenant des groupes hydroxyméthyle est présent en tant qu'un mélange de glycosaminoglycanes non réticulés et réticulés.

7. Glycosaminoglycane contenant des groupes hydroxyméthyle selon l'une quelconque des revendications précédentes destiné à être utilisé selon l'une quelconque des revendications 1 à 6,
**caractérisé en ce**
**qu'**il est présent sous forme de préparation injectable, d'onguent, de crème, de lotion, de gel, de spray, de teinture, de solution de rinçage ou de perfusion, de solution en gouttes, de shampoing, de feuilles occlusives, de poudre ou de préparation inhalable, en particulier d'aérosol.

8. Glycosaminoglycane contenant des groupes hydroxyméthyle selon l'une quelconque des revendications précédentes destiné à être utilisé selon l'une quelconque des revendications 1 à 7,
**caractérisé en ce**
**que** la maladie infectieuse de la peau ou des muqueuses est choisie parmi des maladies de surfaces corporelles, du tractus gastro-intestinal, du genou, du tractus urogénital et des poumons.

9. Glycosaminoglycane contenant des groupes hydroxyméthyle selon l'une quelconque des revendications précédentes destiné à être utilisé selon l'une quelconque des revendications précédentes,
dans la médecine humaine ou vétérinaire.

10. Glycosaminoglycane contenant des groupes hydroxyméthyle selon l'une quelconque des revendications précédentes destiné à être utilisé selon l'une quelconque des revendications précédentes,
**caractérisé en ce**
**que** le glycosaminoglycane contenant des groupes hydroxyméthyle est présent en combinaison avec au moins un inhibiteur de la dégradation de glycosaminoglycane, dans lequel l'inhibiteur de la dégradation de glycosaminoglycane est choisi en particulier parmi l'héparine, l'indométacine, les salicylates, des agents anti-radicaux, tels que la vitamine A, C ou E, et des mélanges de ceux-ci.

11. Glycosaminoglycane contenant des groupes hydroxyméthyle selon l'une quelconque des revendications précédentes destiné à être utilisé selon l'une quelconque des revendications précédentes,
**caractérisé en ce**
**que** le glycosaminoglycane contenant des groupes hydroxyméthyle est présent en combinaison avec un ou plusieurs autres glycosaminoglycanes sous une forme réticulée ou non réticulée, lesquels peuvent contenir en option des groupes hydroxyméthyle, dans lequel les autres glycosaminoglycanes sont choisis en particulier parmi l'héparine, le sulfate de chondroïtine, la chitosamine, la poly-N-acétylglucosamine.

12. Glycosaminoglycane contenant des groupes hydroxyméthyle selon l'une quelconque des revendications précédentes destiné à être utilisé en particulier selon l'une quelconque des revendications précédentes,
**caractérisé en ce**
**que** le glycosaminoglycane contenant des groupes hydroxyméthyle est présent en combinaison avec de la taurolidine.

13. Glycosaminoglycane contenant des groupes hydroxyméthyle selon l'une quelconque des revendications précédentes destiné à être utilisé selon l'une quelconque des revendications précédentes dans le traitement ou la prophylaxie de maladies infectieuses de l'œil, en particulier de la conjonctive ou de la cornée de l'œil, telles qu'une conjonctivite,
dans lequel le glycosaminoglycane contenant des groupes hydroxyméthyle selon l'une quelconque des revendications 1 - 7 est instillé de manière préférée en tant que gel ou est employé en tant que solution de rinçage.

14. Procédé de fabrication d'un glycosaminoglycane contenant des groupes hydroxyméthyle, comprenant les étapes :
(i) de fourniture de glycosaminoglycane, et
(ii) de substitution d'un ou de plusieurs groupes amino du glycosaminoglycane avec des groupes hydroxyméthyle,
dans lequel le glycosaminoglycane est choisi en particulier parmi l'acide hyaluronique, l'héparine, le sulfate de chondroïtine, la chitosamine et la poly-N-acétylglucosamine.

15. Procédé selon la revendication 14,
(a) dans lequel le glycosaminoglycane est isolé lors de l'étape (i) à partir d'une source biologique ou est obtenu de manière biotechnologique, en particulier à partir de crête de coq ou de bactéries transgéniques, telles que *B. subtilis* ou cultures de streptocoques, et/ou
(b) dans lequel l'étape (ii) comprend la mise en réaction du glycosaminoglycane avec du formaldéhyde ou un agent libérant du formaldéhyde dans les conditions de réaction, tel que la taurolidine, et/ou
(c) par ailleurs comprenant l'étape
(iii) d'épuration du glycosaminoglycane substitué par des groupes hydroxyméthyle en particulier par la précipitation avec par exemple des alcools ou des sels, par un procédé de chromatographie, un procédé de dialyse, une extraction sous vide ou une lyophilisation, et/ou
(d) comprenant par ailleurs l'étape
(iv) de réticulation croisée du glycosaminoglycane substitué par des groupes hydroxyméthyle, dans lequel l'étape (iv) comprend en particulier la réticulation croisée avec un ou plusieurs autres glycosaminoglycanes, qui peuvent être modifiés eux-mêmes également sur des groupes amino avec des groupes hydroxyméthyle.
